# EUROPEAN PATENT SPECIFICATION

(11) **EP 1 545 709 B1**
(45) Date of publication and mention of the grant of the patent: **14.11.2007**
(21) Application number: 03750185.5
(22) Date of filing: 02.09.2003
(51) Int. Cl.: A61P 35/00, A61K 31/138, A61K 31/704, A61K 31/337

(54) **NEOADJUVANT TREATMENT OF BREAST CANCER**
PRÄOPERATIVE BEHANDLUNG VON BRUSTKREBS
TRAITEMENT NEOADJUVANT DU CANCER DU SEIN

(30) Priority: 03.09.2002 US 407242 P
(43) Date of publication of application: 29.06.2005
(73) Proprietor: The University of Manitoba, Winnipeg, Manitoba R3T 5V4 (CA); Brandes, Lorne J., Winnipeg Manitoba R3N 1A3 (CA)
(72) Inventor: BRANDES, Lorne, J., Winnipeg, Manitoba R3N 1A3 (CA)
(74) Representative: Smart, Peter John
(86) International application number: PCT/CA2003/001335
(87) International publication number: WO 2004/022163

(56) References cited:
- WO-A-03/037318
- WO-A-03/039526
- MUGGIA F M ET AL: "MODULATION OF TAXANES IN BREAST CANCER" CANCER INVESTIGATION, MARCEL DEKKER INC, US, vol. 18, no. SUPPL 1, 2000, pages 64-66, XP008027009 ISSN: 0735-7907
- MENENDEZ A T ET AL: "MECHANISM OF ACTION OF DPPE, A CHEMOSENSITIZING AGENT" PROCEEDINGS OF THE ANNUAL MEETING OF THE AMERICAN ASSOCIATION FOR CANCER RESEARCH, NEW YORK, NY, US, vol. 39, March 1998 (1998-03), page 509, XP001184767 ISSN: 0197-016X
- KHOO K ET AL: "PHASE II TRIAL OF N,N-DIETHYL-2-Ä4-(PHENYLMETHYL)PHENOXYÜETH ANAMINE. HCL AND DOXORUBICIN CHEMOTHERAPY IN METASTATIC BREAST CANCER: A NATIONAL CANCER INSTITUTE OF CANADA CLINICAL TRIALS GROUP STUDY" JOURNAL OF CLINICAL ONCOLOGY, GRUNE AND STRATTON, NEW YORK, NY, US, vol. 17, no. 11, November 1999 (1999-11), pages 3431-3437, XP001145566 ISSN: 0732-183X
- BRANDES L J ET AL: "THE INTRACELLULAR HISTAMINE ANTAGONIST, N,N-DIETHYL-2-Ä(PHENYLMETHYL) PHENOXYÜ ETHANAMINE. HCL, MAY POTENTIATE DOXORUBICIN IN THE TREATMENT OF METASTATIC BREAST CANCER9 RESULTS OF A PILOT STUDY" BREAST CANCER RESEARCH AND TREATMENT, NIJHOFF, BOSTON, US, vol. 49, no. 1, May 1998 (1998-05), pages 61-68, XP009005592 ISSN: 0167-6806

## Description

### FIELD OF THE INVENTION

The present invention relates to the treatment of breast cancer.

### BACKGROUND OF THE INVENTION

One of the major chemotherapeutic treatments is that of malignant growth (cancer) in humans. The objective of chemotherapy is the total extermination of clonogenic tumor or malignant cells, with minimal damage to the patient. However, one of the major limitations of the chemotherapeutic approach for managing human cancer is the general inability of anticancer drugs to discriminate between normal and tumorous cells. Anti-neoplastic agents have the lowest therapeutic indicies of any class of drugs used in humans and hence produce significant and potentially life-threatening toxicities. Certain commonly-used anti-neoplastic agents have unique and acute toxicities for specific tissues. For example, the vinca alkaloids possess significant toxicity for nervous tissues, while adriamycin has specific toxicity for heart tissue and bleomycin has for lung tissue. In general, almost all members of the major categories of anti-neoplastic agents have considerable toxicities for normal cells of gastrointestinal, epidermal and myelopoietic tissues.

Generally, the dose-limiting consideration for chemical management of cancer in humans is the toxicity that anti-neoplastic agents have for the pluripotent stem cells of myelopoietic tissue. This toxicity arises from the fact that most anticancer drugs function preferentially against proliferating cells but with no significant capacity to discriminate between cycling normal and cycling tumor tissues.

In certain types of locally-advanced breast cancer, specifically inflammatory or T3 to T4 breast cancer, there is applied a treatment with chemotherapeutic agents prior to surgical removal of the tumor, in order to reduce the size of tumor. T3 tumors are tumors sized >3 and <4 cm. T3 tumors may be operable or inoperable depending on where in the breast they are located. For example, they are often inoperable if close to the chest wall, especially in small breasts. T4 tumors are tumors sized >4 cm and generally are inoperable. Inflammatory breast cancer infiltrates the lymphatics of the skin, is usually a diffuse tumor and very high grade in term of malignancy.

In US Patents Nos. 6,288,799, 5,859,065, 5,708,329, 5,747,543 and 5,618,846, all assigned to University of Manitoba, there is described an improved method for the *in vivo* chemotherapeutic treatment of cancer in which there is first administered a compound which inhibits normal cell proliferation while promoting malignant cell proliferation, specifically a potent antagonist selective for intracellular histamine receptors, in an amount sufficient to inhibit the binding of intracellular histamine to the receptors in normal and malignant cells. Following sufficient time to permit the inhibition of binding of intracellular histamine, a chemotherapeutic agent is administered. An enhanced toxic effect on the cancer cells from the chemotherapeutic agent is obtained while any adverse effect of the chemotherapeutic agent on normal cells, particularly bone marrow and gastro-intestinal cells, is significantly ameliorated. One useful compound which inhibits normal cell proliferation while promoting malignant cell proliferation is N,N-diethyl-2-[4-(phenylmethyl)-phenoxy]ethanamine, abbreviated herein as DPPE.

### SUMMARY OF INVENTION

It has now surprisingly been found, in a Phase II clinical trial, the procedure described in the aforementioned patents when using a combination of an anthracycline chemotherapeutic agent and a taxane therapeutic agent is an effective procedure in neoadjuvant treatment of inflammatory breast cancer or T3 to T4 breast cancer. In addition, the procedure leads to long term survival post surgery.

Accordingly, the present invention provides a use of at least one diphenyl compound of the formula: 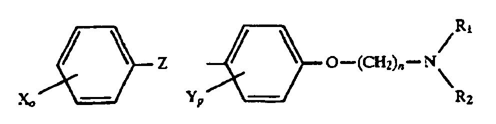 wherein X and Y are each fluorine, chlorine or bromine, Z is an alkylene group of 1 to 3 carbon atoms or =C=O, or the phenyl groups are joined to form a tricyclic ring, o and p are 0 or 1, R₁ and R₂ are each an alkyl group containing 1 to 3 carbon atoms or are joined together to form a heterocyclic ring with the nitrogen atom and n is 1, 2 or 3, or pharmaceutically-acceptable salts thereof, and the combination of an anthracycline chemotherapeutic agent and a taxane chemotherapeutic agent in the manufacture of a medicament for the neoadjuvant chemotherapy of human patients with inflammatory breast cancer or T3 or T4 breast cancer by subjecting the patients to a plurality of cycles of chemotherapy at predetermined intervals until cancerous tissue is reduced to an operable size or is in remission, each cycle comprising:
(a) first administering to the patient the at least one diphenyl compound, and
(b) following sufficient time to permit inhibition of binding of intracellular histamine, subsequently administering to the patient the combination of chemotherapeutic agents.

In the application of the present invention, the diphenyl compound and the chemotherapeutic agents are generally administered by intravenous infusion. In one preferred procedure, a solution of the diphenyl compound is administered to the patient over a desired period of time prior to administration of the chemotherapeutic agents and a solution of the chemotherapeutic agents in combination with the diphenyl compound then is administered for the period of administration of the chemotherapeutic agents. If desired, a solution of the diphenyl compound is administered after completion of the administration of the chemotherapeutic agents for a desired period of time to ameliorate side effects from the administration of the chemotherapeutic agents.

### GENERAL DESCRIPTION OF INVENTION

In the present invention, a diphenyl compound is used which is a potent antagonist of histamine binding at the intracellular histamine receptor and is administered in an amount sufficient to inhibit the binding of intracellular histamine at the intracellular binding site (H_{IC}) in normal cells. Such compounds exhibit a pKi of at least about 5, preferably at least about 5.5.

Specific potent compounds which are useful in the present invention are diphenyl compounds of the formula: 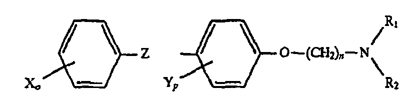 wherein X and Y are each fluorine, chlorine or bromine, Z is an alkylene group of 1 to 3 carbon atoms or =C=O, o and p are 0 or 1, R₁ and R₂ are each alkyl groups containing 1 to 3 carbon atoms or are joined together to form a hetero-ring with the nitrogen atom and n is 1, 2 or 3. Pharmaceutically-acceptable salts of the diphenyl compounds may be employed.

Alternatively, the benzene rings may be joined to form a tricyclic ring, in accordance with the structure:

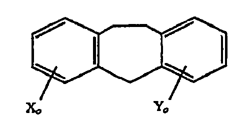

In one preferred embodiment, the group 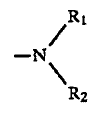 is a diethylamino group, although other alkylamino groups may be employed, such as dimethylamino, and, in another preferred embodiment, a morpholino group, although other heterocyclic ring groups may be employed, such as piperazino. o and p are usually 0 when Z is an alkylene group and n may be 2. In one particularly preferred embodiment, Z is -CH₂-, n is 2, o and p are each 0 and 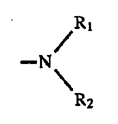 is a diethylamino group. This compound, namely N,N-diethyl-2-[4-(phenylmethyl)-phenoxy]ethanamine, which may be in the form of the free base or in the form of its hydrochloride or other pharmaceutically-acceptable salt, is abbreviated herein as DPPE. In addition to a methylene group linking the benzene rings, other linking groups may be employed, such as =C=O. Other substitutents may be provided on the benzene rings in addition to the halogen atoms, for example; an imidazole group.

The diphenyl compound employed in the present invention is administered to the patient in any convenient manner, such as by intravenous injection of a solution thereof in an aqueous pharmaceutically-acceptable vehicle. The diphenyl compound is administered to the patient over a period of time before administration of the chemotherapeutic agents.

The chemotherapeutic agents employed herein are anthracyclines, preferably doxorubicin and epirubicin; and taxanes, preferably Taxol (Trademark of Bristol-Myers Squibb for paclitaxel) and Taxotere (Trademark of Aventis Pharma for docetaxel). The mixture of chemotherapeutic agents is administered in any manner consistent with their normal manner of administration in conventional breast cancer therapy, usually by intravenous infusion of a solution thereof.

The administration of the diphenyl compound to the patient prior to administration of the chemotherapeutic agents is necessary in order to permit the diphenyl compound to inhibit the binding of intracellular histamine in normal and malignant cells and thereby, in effect, shut down the proliferation of the normal cells, but increase proliferation of malignant cells.

The length of time prior to administration of the chemotherapeutic agents that the diphenyl compound is administered depends on the diphenyl compound, its mode of administration and the size of the patient. Generally, the diphenyl compound is administered to the patient for about 30 to about 90 minutes, preferably about 60 minutes, prior to administration of the chemotherapeutic agents.

The quantity of diphenyl compound administered to the patient depends on the side effects to be ameliorated, but should be at least sufficient to inhibit binding of intracellular histamine in normal cells. The quantity required to achieve the beneficial effects of the present invention depends upon the diphenyl compound employed, the chemotherapeutic agents employed and the quantity of such agents employed.

In general, the quantity of diphenyl compound employed in humans is from about 8 to about 320 mg/M² of human to which the diphenyl compound is administered, with about 8 and 240 mg/M² being the optimal dose for gastro-intestinal and bone marrow protection, respectively. Over this dose range, the present invention is able to achieve an enhanced chemotherapeutic effect on breast cancer cells while, at the same time, also protecting normal cells from damage by the chemotherapeutic agents in a wide variety of circumstances where traditional chemotherapy leads to damage of normal cells or tissues not involved in the disease process.

In the neoadjuvant treatment of inflammatory or T3 to T4 breast cancer, the diphenyl compound preferably is used in an amount of about 3 to about 10 mg/kg of patient, administered intravenously over a period of about 30 to about 90 minutes prior to administration of the chemotherapeutic agents and continuing for the period of administration of the chemotherapy agent. In the specific Phase II clinical trial described herein, there was employed 6 mg/kg of DPPE in the form of its hydrochloride salt, administered intravenously as an aqueous solution thereof over 80 minutes, with the last twenty minutes being accompanied by infusion of the chemotherapeutic agents, followed by the intravenous administration of an aqueous solution at a dose of 2.5 mg/kg of DPPE for 180 minutes accompanied by the infusion of Taxol or for 60 minutes accompanied by the infusion of Taxotere.

A second regimen for DPPE/Taxotere treatment is the intravenous administration of an aqueous solution of DPPE for 80 minutes, with the last 20 minutes being accompanied by infusion of the Taxotere, followed by infusion of Taxotere alone for 40 minutes.

The chemotherapy agents which are employed herein preferably are used in a total amount of 75 to about 225 mg/M² of patient consistent with the identity of the chemotherapy agent. The chemotherapeutic agents may be administered in an amount of about 50 to about 60 mg/M² of patient for doxorubicin or epirubicin, about 175 to about 225 mg/M² of Taxol and about 75 to about 100 mg/M² of Taxotere. In the specific Phase II clinical trial described herein, there was employed 50 mg/M² of doxorubicin or epirubicin, and 175 mg/M² of Taxol or 75 mg/M² of Taxotere, administered over the last 20 minutes of infusion of the DPPE solution and over a further 180 minutes for Taxol or 60 minutes for Taxotere, accompanied by infusion of a 2.5 mg/kg of DPPE solution.

As noted above, patients with inflammatory breast cancer or T3 to T4 breast cancer are subjected to a number of cycles of chemotherapy at predetermined intervals to reduce the size of the tumor to an operable size. The number of cycles for each patient is generally about 5 to about 8 cycles, with about 21 to about 28 days between each cycle. In the Phase II clinical trial, patients were subjected to 6 cycles at time intervals of 21 days.

As set forth herein, a Phase II clinical trial was conducted on patients having inflammatory or T3 to T4 breast cancer in which patients were administered DPPE followed by doxorubicin or epirubicin and Taxol or Taxotere. Various data from the clinical trial were collected and analyzed.

The results of this trial showed that DPPE along with doxorubicin/epirubicin and Taxol/Taxotere was an effective neoadjuvant treatment which lead to long term survival post surgery.

### EXAMPLE

This Example illustrates the neoadjuvant treatment of inflammatory or T3-T4 breast cancer.

A Phase II clinical trial was carried out in which patients (N=8) with inflammatory (N=7) and T3 to T4 (N=1) breast cancer were treated with a combination of DPPE and epirubicin (EPI)/Taxol (N=5), a combination of DPPE and doxorubicin (DOX)/Taxol (N=2) and DPPE and a combination of DPPE and epirubicin/Taxotere (N=1). DPPE was administered at a dose of 6 mg/M² over 80 minutes with a combination of epirubicin or doxorubicin at a dose of 50 mg/M² and Taxol at a dose of 175 mg/M² or Taxotere at a dose of 75 mg/M² over the last 20 minutes and during a further 180 minutes for Taxol or 60 minutes for Taxotere, at a dose of 2.5 mg/kg. The treatment was repeated at 21 day intervals for 6 cycles. The eight patients with inflammatory or T3 to T4 breast cancer had no previous chemo- or radiotherapy. When the chemotherapy cycles were complete, the cancerous tissue was removed and the patients observed.

The results obtained are shown in Table, I. In this Table, the abbreviation TTP stands for time to progression and the abbreviation OS stands for overall survival. Two long-term survivors (45+ and 53+ months) had mixed high-grade tumors pretreatment but no high-grade component post-surgery. A third patient with only high-grade cells or cytology, had a clinical/pathological complete remission. This patient remains disease-free at 55+ months.

These findings are compatible with the hypothesis advanced in copending United States Applications Nos. 60/331,242 filed November 9, 2001 in the names of Lome Brandes and Mark Vincent, that DPPE-based chemotherapy may preferentially target high-grade malignant cell populations.

### SUMMARY OF INVENTION

In summary of this disclosure, the present invention provides a neoadjuvant chemotherapeutic treatment of inflammatory or T3 to T4 breast cancer.

**TABLE I**

| **SUMMARY OF RESULTS NEOADJUVANT THERAPY** | | | | | | |
|---|---|---|---|---|---|---|
| No. Patients | Cycles (Total) | Response* | | | TTP | OS |
| | | | | | (Median, mos.) | |
| | | CR | PR | NR | | |
| 8^{†} | 46 | 4/2* | 3/4* | 1/2* | 21 | 43+ |

| | | | | | | |
|---|---|---|---|---|---|---|
| ^{†}5 DPPE/EPI/TAXOL; 2 DPPE/DOX/TAXOL; 1 DPPE/EPI/TAXOTERE * Pathological criteria | | | | | | |

## Claims

1. The use of at least one diphenyl compound of the formula: 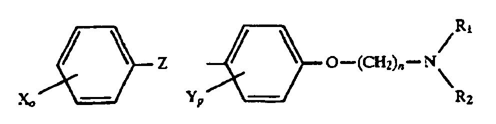 wherein X and Y are each fluorine, chlorine or bromine, Z is an alkylene group of 1 to 3 carbon atoms or =C=O, or the phenyl groups are joined to form a tricyclic ring, o and p are 0 or 1, R₁ and R₂ are each an alkyl group containing 1 to 3 carbon atoms or are joined together to form a heterocyclic ring with the nitrogen atom and n is 1, 2 or 3, or pharmaceutically-acceptable salts thereof, and the combination of an anthracycline chemotherapeutic agent and a taxane chemotherapeutic agent in the manufacture of a medicament for the neoadjuvant chemotherapy of human patients with inflammatory breast cancer or T3 or T4 breast cancer by subjecting the patients to a plurality of cycles of chemotherapy at predetermined intervals until cancerous tissue is reduced to an operable size or is in remission, each cycle comprising:
(a) first administering to the patient the at least one diphenyl compound, and
(b) following sufficient time to permit inhibition of binding of intracellular histamine, subsequently administering to the patient the combination of chemotherapeutic agents.

2. The use claimed in claim 1, wherein the group 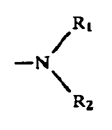 is a diethylamino group, a dimethylamino group, a morpholino group or a piperazino group, preferably a diethylamino group, in which case Z is -CH₂, n is 2 and o and p are each 0, optionally in the form of a hydrochloride salt.

3. The use claimed in claim 1 or 2, wherein said anthracycline chemotherapeutic agent is doxorubicin or epirubicin.

4. The use claimed in any one of claims 1 to 3, wherein said taxane chemotherapeutic agent is Taxol or Taxotere.

5. The use claimed in any one of claims 1 to 4, wherein said diphenyl compound is administered to the patient 30 to 90 minutes, preferably 60 minutes, prior to said administration of said combination of chemotherapeutic agents, preferably by intravenous infusion of a solution of the diphenyl compound over a period of time of up to 90 minutes prior to administration of said chemotherapeutic agents and is maintained during administration of said combination of chemotherapeutic agents, more preferably by administering said diphenyl compound for 60 minutes prior to administration of said combination of chemotherapeutic agents and is maintaining said administration during intravenous infusion of said combination of chemotherapeutic agents.

6. The use claimed in claim 5, wherein administration of taxane chemotherapeutic agent, optionally in combination with anthracycline chemotherapeutic agent, is effected during 20 minutes maintenance of infusion of the diphenyl compound, followed by continued infusion of diphenyl compound for the remainder of the administration of the taxane chemotherapeutic agent.

7. The use claimed in any one of claims 1 to 6, wherein said diphenyl compound is administered in an amount of 8 to 240 mg/M² of said patient, preferably 3 to 10 mg/kg of patient, more preferably 6 mg/kg.

8. The use claimed in any one of claims 1 to 7, wherein said combination of chemotherapeutic agents are administered in an amount of 50 to 60 mg/M², preferably 50 mg/M², of patient for doxorubicin or epirubicin, 175 to 225 mg/M², preferably 175 mg/M², for Taxol and 75 to 100 mg/M², preferably 75 mg/M², for Taxotere.

9. The use claimed in any one of claims 1 to 8, wherein the number of cycles of chemotherapy treatment is 5 to 10 administered at intervals of 21 to 28 days.

## Patentansprüche

1. Verwendung von mindestens einer Diphenylverbindung der Formel 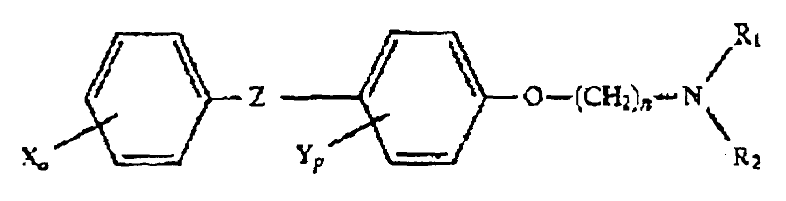 wobei X und Y jeweils Fluor, Chlor oder Brom sind, Z eine Alkylengruppe aus 1 bis 3 Kohlenstoffatomen oder =C=O ist oder die Phenylgruppen so verbunden sind, dass ein Dreiringsystem gebildet wird, o und p 0 oder 1 sind, R₁ und R₂ jeweils eine Alkylgruppe sind, die 1 bis 3 Kohlenstoffatome enthält, oder so miteinander verbunden sind, dass ein Heterozyklus mit dem Stickstoffatom gebildet wird, und n 1, 2 oder 3 ist, oder pharmazeutisch annehmbare Salze davon, und der Kombination eines chemotherapeutischen Anthracyclin-Wirkstoffs und eines chemotherapeutischen Taxan-Wirkstoffs bei der Herstellung eines Medikaments zur neoadjuvanten Chemotherapie menschlicher Patienten mit inflammatorischem Brustkrebs oder T3- oder T4-Brustkrebs indem die Patienten einer Mehrzahl von Chemotherapie-Zyklen in zuvor festgelegten Intervallen unterzogen werden bis das Krebsgewebe auf eine operierbare Größe reduziert ist oder sich im Rückzug befindet, wobei jeder Zyklus umfasst:
(a) zunächst Verabreichen der mindestens einen Diphenylverbindung an den Patienten, und
(b) gefolgt von ausreichend Zeit, um die Inhibition der Bindung intrazellulären Histamins zu ermöglichen, anschließend Verabreichen der Kombination chemotherapeutischer Wirkstoffe an den Patienten.

2. Verwendung gemäß Anspruch 1, wobei die Gruppe 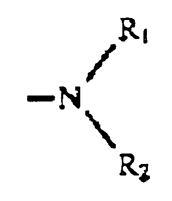 eine Diethylaminogruppe, eine Dimethylaminogruppe, eine Morpholingruppe oder eine Piperazingruppe ist, vorzugsweise eine Diethylaminogruppe, wobei hier Z -CH₂ ist, n 2 ist und o und p jeweils 0 sind, gegebenenfalls in Form eines Hydrochlorid-Salzes.

3. Verwendung wie in Anspruch 1 oder 2 beansprucht, wobei besagter chemotherapeutischer Anthracyclin-Wirkstoff Doxorubicin oder Epirubicin ist.

4. Verwendung wie in einem der Ansprüche 1 bis 3 beansprucht, wobei besagter chemotherapeutischer Taxan-Wirkstoff Taxol oder Taxoter ist.

5. Verwendung wie in einem der Ansprüche 1 bis 4 beansprucht, wobei besagte Diphenylverbindung dem Patienten 30 bis 90 Minuten, vorzugsweise 60 Minuten, vor besagter Verabreichung von besagter Kombination chemotherapeutischer Wirkstoffe verabreicht wird, vorzugsweise durch intravenöse Infusion einer Lösung der Diphenylverbindung über einen Zeitraum von bis zu 90 Minuten vor der Verabreichung besagter chemotherapeutischer Wirkstoffe und während die Verabreichung besagter Kombination chemotherapeutischer Wirkstoffe beibehalten wird, weiter bevorzugt durch Verabreichen besagter Diphenylverbindung für 60 Minuten vor dem Verabreichen besagter Kombinationen chemotherapeutischer Wirkstoffe und wobei besagte Verabreichung während der intravenösen Infusion von besagter Kombination chemotherapeutischer Wirkstoffe beibehalten wird.

6. Verwendung wie in Anspruch 5 beansprucht, wobei die Verabreichung von chemotherapeutischem Taxan-Wirkstoff, gegebenenfalls in Kombination mit chemotherapeutischem Anthracyclin-Wirkstoff, in 20 Minuten unter Beibehaltung der Infusion der Diphenylverbindung erfolgt, gefolgt von der andauernden Infusion der Diphenylverbindung für den Rest der Verabreichung des chemotherapeutischen Taxan-Wirkstoffs.

7. Verwendung wie in einem der Ansprüche 1 bis 6 beansprucht, wobei besagte Diphenylverbindung in einer Menge von 8 bis 240 mg/M² von besagtem Patienten, vorzugsweise von 3 bis 10 mg/kg des Patienten, weiter bevorzugt von 6 mg/kg verabreicht wird.

8. Verwendung wie in einem der Ansprüche 1 bis 7 beansprucht, wobei besagte Kombination chemotherapeutischer Wirkstoffe in einer Menge von 50 bis 60 mg/M², vorzugsweise 50 mg/M² des Patienten für Doxorubicin und Epirubicin, 175 bis 225 mg/M², vorzugsweise 175 mg/M² für Taxol und 75 bis 100 mg/M², vorzugsweise 75 mg/M² für Taxoter verabreicht werden.

9. Verwendung wie in einem der Ansprüche 1 bis 8 beansprucht, wobei die Anzahl an Zyklen der chemotherapeutischen Behandlung 5 bis 10 beträgt, die in Intervallen von 21 bis 28 Tagen verabreicht werden.

## Revendications

1. Utilisation d'au moins un composé de type diphényle de formule : 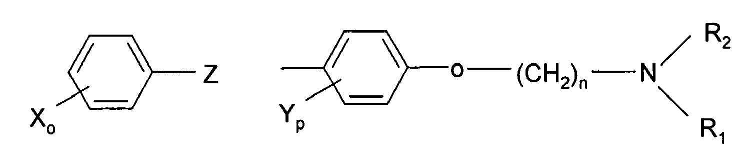 où X et Y sont chacun le fluor, le chlore ou le brome, Z est un groupe alkylène de 1 à 3 atomes de carbone ou =C=O, ou bien les groupes phényle sont joints pour former un cycle tricyclique, o et p sont 0 ou 1, R₁ et R₂ sont chacun un groupe alkyle contenant 1 à 3 atomes de carbone ou sont joints entre eux pour former un cycle hétérocyclique avec l'atome d'azote et n est 1, 2 ou 3, ou de ses sels pharmaceutiquement acceptables, et de la combinaison d'un agent chimiothérapeutique de type anthracycline et d'un agent chimiothérapeutique de type taxane dans la fabrication d'un médicament pour la chimiothérapie néo-adjuvante de patients humains ayant un cancer du sein inflammatoire ou un cancer du sein T3 ou T4 par exposition des patients à une pluralité de cycles de chimiothérapie à des intervalles prédéterminés jusqu'à ce que le tissu cancéreux soit réduit à une taille opérable ou soit en rémission, chaque cycle comprenant :
(a) une première administration au patient d'au moins un composé de type diphényle, et
(b) après une durée suffisante pour permettre l'inhibition de la liaison de l'histamine intracellulaire, une administration subséquente au patient de la combinaison d'agents chimiothérapeutiques.

2. Utilisation selon la revendication 1 où le groupe 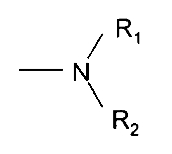 est un groupe diéthylamino, un groupe diméthylamino, un groupe morpholino ou un groupe pipérazino, de préférence un groupe diéthylamino, auquel cas Z est -CH₂, n est 2 et o et p sont chacun 0, éventuellemt sous forme d'un sel chlorhydrate.

3. Utilisation selon la revendication 1 ou 2 où ledit agent chimiothérapeutique de type anthracycline est la doxorubicine ou l'épirubicine.

4. Utilisation selon l'une quelconque des revendications 1 à 3 où ledit agent chimiothérapeutique de type taxane est le Taxol ou le Taxotère.

5. Utilisation selon l'une quelconque des revendications 1 à 4 où ledit composé de type diphényle est administré au patient 30 à 90 minutes, de préférence 60 minutes, avant ladite administration de ladite combinaison d'agents chimiothérapeutiques, de préférence par perfusion intraveineuse d'une solution du composé de type diphényle sur une durée pouvant atteindre 90 minutes avant l'administration desdits agents chimiothérapeutiques et est maintenu pendant l'administration de ladite combinaison d'agents chimiothérapeutiques, de préférence encore par administration dudit composé de type diphényle pendant 60 minutes avant l'administration de ladite combinaison d'agents chimiothérapeutiques et maintien de ladite administration pendant la perfusion intraveineuse de ladite combinaison d'agents chimiothérapeutiques.

6. Utilisation selon la revendication 5 où l'administration d'un agent chimiothérapeutique de type taxane, éventuellement en combinaison avec un agent chimiothérapeutique de type antracycline, est réalisée pendant un maintien de 20 minutes de la perfusion du composé de type diphényle, suivi par la perfusion prolongée du composé de type diphényle pendant le reste de l'administration de l'agent chimiothérapeutique de type taxane.

7. Utilisation selon l'une quelconque des revendications 1 à 6 où ledit composé de type diphényle est administré en une quantité de 8 à 240 mg/M² dudit patient, de préférence de 3 à 10 mg/kg du patient, de préférence encore 6 mg/kg.

8. Utilisation selon l'une quelconque des revendications 1 à 7 où ladite combinaison d'agents chimiothérapeutiques est administrée en une quantité de 50 à 60 mg/M², de préférence 50 mg/M², du patient pour la doxorubicine ou l'épirubicine, 175 à 225 mg/M², de préférence 175 mg/M², pour le Taxol et 75 à 100 mg/M², de préférence 75 mg/M², pour le Taxotère.

9. Utilisation selon l'une quelconque des revendications 1 à 8 où le nombre de cycles de traitement chimiothérapeutique est 5 à 10 administrés à des intervalles de 21 à 28 jours.
